# EUROPEAN PATENT SPECIFICATION

(11) **EP 1 448 207 B1**
(45) Date of publication and mention of the grant of the patent: **27.04.2005**
(21) Application number: 02783334.2
(22) Date of filing: 26.11.2002
(51) Int. Cl.: A61K 31/565

(54) **DOSAGE REGIMEN AND PHARMACEUTICAL COMPOSITION FOR EMERGENCY CONTRACEPTION**
BEHANDLUNGSVERFAHREN UND PHARMAZEUTISCHE ZUSAMMENSETZUNG ZUR NOTFALL-EMPFÄNGNISVERHÜTUNG
POSOLOGIE ET COMPOSITION PHARMACEUTIQUE POUR CONTRACEPTION D'URGENCE

(30) Priority: 27.11.2001 HU 0105173
(43) Date of publication of application: 25.08.2004
(73) Proprietor: RICHTER GEDEON VEGYESZETI GYAR RT., 1103 Budapest (HU)
(72) Inventor: VAN LOOK, Paul, F., A., CH-1211 Geneva 27 (CH); BALOGH, Illésné, H-1222 Budapest (HU); KOMANDI, Katalin, H-1111 Budapest (HU); NEMES, Lászlo, H-1141 Budapest (HU); SZABO, Zsolt, H-2141 Csömör (HU)
(74) Representative: HOFFMANN EITLE
(86) International application number: PCT/HU2002/000129
(87) International publication number: WO 2003/045397

(56) References cited:
- OF FERTILITY REGULATION T F O P M: "Randomised controlled trial of levonorgestrel versus the Yuzpe regimen of combined oral contraceptives for emergency contraception" LANCET, XX, XX, vol. 352, no. 9126, 8 August 1998 (1998-08-08), pages 428-433, XP004265510 ISSN: 0140-6736
- VON HERTZEN H ET AL: "Low dose mifepristone and two regimens of levonorgestrel for emergency contraception: a WHO multicentre randomised trial" LANCET, XX, XX, vol. 360, no. 9348, 7 December 2002 (2002-12-07), pages 1803-1810, XP004397554 ISSN: 0140-6736

## Description

This invention relates to a dosage regimen for emergency contraception, to pharmaceutical compositions of the same purpose, the use of levonorgestrel for the manufacture of pharmaceutical compositions for the same purpose, as well as the process of manufacturing these pharmaceutical compositions.

It is known that coitus takes place in numerous cases, when conception must be prevented by all means. Several devices and pharmaceutical compositions are available for the prevention of undesirable conception in the case of regular and planned coitus. For example, condom, pessary, intrauterine devices as well as the different mono-or multi-phasic oral contraceptives.

But in the case of non-planned coitus or coitus with imperfect contraception these devices are not available or only in inadequate way, therefore precautions for preventing conception must be taken after the coitus. Such is the situation for example in the case of women, who have non-regular sexual life or the victims of rapes, or if the device for preventing conception, for example the condom, gets damaged or torn. Since in these cases the contraception must be achieved before the implantation of the fertilized egg- in relatively short time - these methods are called emergency contraception.

Norgestrel and levonorgestrel, namely the D isomer of norgestrel [±-17α-13-ethyl-17-hydroxy-18,19-dinorpregn-4-en-20-in-3-on] have been used in combined preparations as contraceptives for a long time. For example a pharmaceutical composition called OVRAL, which appeared in 1968 and contained 0.05 mg of ethynyl-estradiol and 0.5 mg of norgestrel. The British patent No 1,041,280 describes the preparation of tablets, capsules and suspension of anabolic activity containing 5 mg of norgestrel as active ingredient, although in the description of the biological activity of the active ingredient the estrogen antagonist activity is said to be 53.7-fold compared to that of the progesterone and the progestogen activity is the same as that of the progesterone.

In the 1970's comprehensive studies were carried out [The Journal of Reproductive Medicine, **13(2)**, (1974); Contraception, **7(5)**, 367-379, (1973); Reproduction, **2(1)**, 61-62, (1975); International Journal of Fertility, **20**, 156-160, (1975)] to test the use of several progestins, among others levonorgestrel, as routine postcoital contraceptives. Routine postcoital contraception means that women, who have non-regular sexual life, use the medicine in a planned way, but after the coitus. The single daily doses used were between 150 µg and 1000 µg. The results of the studies showed that the postcoital contraceptive efficacy of levonorgestrel ― if it is used alone ― was low even in 1 mg dose.

A. A. Yuzpe and his coworkers reported in The Journal of Reproductive Medicine [**13(2)**, (1974)] the results of those studies where a pharmaceutical composition containing 100 µg of ethinyl-estradiol and 1.0 mg of norgestrel was used as postcoital contraceptive in a single dose. The composition was administered within five days of the unprotected coitus. Later the method was modified. On the one hand the period of the possible use of the composition was reduced from 5 days to 72 hours, on the other hand the dose was doubled that way that the administration was repeated 12 hours after the first one. [Fertility and Sterility, **28**, 932-936, (1977), ibid. **37**, 508-513 (1982); International Journal of Gynaecology and Obstetrics, **15**, 133-136, (1977)]. This modification increased the success of the method.

After the studies of A. A. Yuzpe and his co-workers several other trials were carried out to prove the efficacy of this combination. In these studies the total dose of ethinyl-estradiol was 0.2 mg combined with 2.0 mg of norgestrel or 1.0 mg of levonorgestrel. The results of the studies showed that although the above administration (Yuzpe regimen) caused less side-effects than the estrogens used earlier in high doses (mainly in the sixties), the relative incidence of nausea and vomiting was still very high (50 and 20 %, respectively). These side-effects are due to the estrogen effect and they cause the decrease in compliance, moreover, if vomiting occurs, it decreases the efficacy of the treatment.

The use of levonorgestrel in emergency contraception was discovered in the 1990's. The results of the studies were reported in two well-documented publications [Lancet, **352**, 428-433, (1998) and Human Reproduction, **8(3)**, 389-392, (1993)]. The efficacy of tablets containing only 0.75 mg of levonorgestrel and the combined tablets of Yuzpe method containing 0.1 mg of ethinyl-estradiol + 1.0 mg levonorgestrel were studied by administering the doses 12 hours apart within 48 as well as within 72 hours of unprotected coitus. The results showed that protection with two tablets containing 0.75 mg of levonorgestrel was better than with the Yuzpe regimen, but the women, who received only levonorgestrel, observed less side-effects, which could be due to the lack of ethinyl-estradiol.

The mechanism of action of levonorgestrel used as postcoital contraceptive was investigated in several studies. Keserü and Garmendia showed that the antiovulatory effect probably depends partly on the time elapsed between taking the last tablet and the time of ovulation, partly on the quantity of the applied hormone [Contraception, **10**, (1974)]. According to other authors besides the inhibition of ovulation other factors can also influence the contraceptive effect [Contraception, **63**, 123-129, (2001)]. Levonorgestrel administered in the follicular phase decreased the proliferation activity of the endometrium, while in the luteal phase there was no effect [Contraception, **39(3)**, 275-289, (1989)].

Several trials were conducted to show the effect of levonorgestrel on the cervical mucus, which could be observed a few hours after the administration. Levonorgestrel inhibits the sperms getting into the upper genital tract in such a way that it causes the thickening of the cervical mucus almost immediately after the absorption of the hormone. It was also shown that after the administration of 400 µg of levonorgestrel the alkalization of the intrauterine fluid starts already after 4 hours of administration and it lasts for approximately 48 hours. This effect can play a role in the inhibition of the movement of sperms, consequently in the contraceptive effect as well [Contraception, **11(1)**, (1975)].

The studies showed that two pharmaceutical compositions containing 0.75-0.75 mg of levonorgestrel used at 12 hours' interval within 72 hours after the unprotected coitus successfully inhibited the conceptions which otherwise might have occurred. The efficacy was significantly better than the efficacy of the Yuzpe regimen used worldwide earlier. Because of the lack of the estrogen component, side effects (nausea, feeling of sickness, vomiting) leading to the decrease in compliance and the efficacy of the treatment were observed far less frequently. The results of the clinical studies showed that the efficacy was the better the earlier the treatment started after the coitus. However, according to experience, if women wanted to follow the instructions correctly, they often delayed taking the first tablet so as taking the second dose after 12 hours would not fall on an extremely inconvenient time (for example 3 o'clock in the morning). The results of the studies showed that the prescription of 12 hours' interval between the two doses decreased the compliance. According to statistical data the majority of women took the second dose within 12 to 16 hours after the first one [Lancet, **352**, 428-433, (1998)].

The results of the study of the mechanism of action of levonorgestrel used as postcoital contraceptive showed that the anti-ovulatory effect had great importance. According to the literature [Contraception, **63**, 123-129, (2001)] the anti-ovulatory effect is appr. 42 % of the total effect, while the rest is distributed among the effects on the cervical mucus, the migration of sperms, zygote transport through the fallopian tube, the endometrium and the implantation. The anti-ovulatory effect depends partly on the quantity of the applied hormone, partly on the time elapsed between the administration and the expected time of ovulation. This seems to support the importance of the 12-hour interval.

According to the above mentioned facts, taking 0.75 mg of levonorgestrel for emergency contraception twice at 12-hour interval within 72 hours of the coitus seemed to be reasonable. Despite of this we studied the possibility of applying the two doses at the same time within 72 hours of the unprotected coitus so as to eliminate the disadvantages of the 12-hour interval. Surprisingly it was found that the administration of two doses of 0.75 mg of levonorgestrel as an active ingredient at the same time did not cause a decrease in efficacy.

Therefore the object of this invention is a dosage regimen for emergency contraception and a pharmaceutical composition for the application of this regimen. The regimen according to this invention is that a single dose containing only 1.5 ± 0.2 mg of levonorgestrel as active ingredient is administered to women to be treated with a pharmaceutical composition within 72 hours of unprotected coitus. Further objects of this invention are the dosage units required to carry out the above regimen.

The dosage units required to carry out the above regimen can be in solid or liquid state, and they can be for example tablets, film-coated tablets, coated tablets, capsules, pills or powder preparations. The lyophilized powder ampoule preparations - making the in situ preparation of liquid compositions possible - also belong to them. Liquid compositions can be for example solutions for injection or infusion.

The efficacy of the regimen according to this invention was compared with the known regimen administering 0.75-0.75 mg of levonorgestrel 12 hours apart in the following trials.

In a double-blind, randomized, multicentre, multinational trial 1,356 women received 0.75-0.75 mg of levonorgestrel at 12-hour interval in the traditional way, while 1,356 received in a single dose 1.5 ± 0.2 mg of levonorgestrel within 72 hours of the unprotected coitus. In the group treated in the traditional way the conception rate was 1.77 %, while in the other group treated with a single dose it was 1.47 %. The so-called "Prevented fraction" (this number shows how many percent of the conception which otherwise might happen is prevented by the treatment) was 77.3 % in the group treated in the traditional way, while in the other it was 81.9 %. These data support the advantage of the single dose treatment, although statistical significance can not be proven. The results of the trial proved that the conceptions which otherwise might have happened could be prevented by a single tablet containing twice 0.75 mg of levonorgestrel at least as effectively as by the known therapy.

During the evaluation of undesired effects it was found that the incidence of nausea was 14.5 % in the traditional group, while in the group treated with a single dose it was 13.7 %. The incidence of vomiting was 1.4 % in both groups. As the new regimen also has the advantageous properties resulting from the lack of the estrogen component, the incidence of nausea, feeling of sickness, vomiting was apparently low. The incidence of other side-effects (diarrhoea, fatigue, dizziness, headache, breast tenderness, lower abdominal pain) was also not differing significantly.

The incidence of menstrual disorders was not increased either by the single administration of 1.5 mg dose of levonorgestrel compared to the administration of two 0.75 mg doses. The incidence of menstrual disorders was 30.9 % in both groups.

The results of the comparative study show that a single dose of 1.5 ± 0.2 mg of levonorgestrel surprisingly prevented the conception which otherwise might have occurred with the same success as if this amount of active ingredient were administered twice at 12-hour interval within 72 hours of the unprotected coitus. However, the single administration increases the compliance and decreases the possibility of incorrect use by women . Regarding side-effects it has the same advantageous properties as the known double dosed composition containing only levonorgestrel for emergency contraception.

The invention is illustrated by the following not limiting Examples:

### Example 1

Tablets of 100 mg total weight are prepared from the following ingredients for each tablet:

| | |
|---|---|
| levonorgestrel | 1.5 mg |
| hydrophilic colloidal silicium dioxide | 0.5 mg |
| maize-starch | 23.5 mg |
| potato-starch | 0.5 mg |
| talcum | 2.5 mg |
| magnesium stearate | 1.0 mg |
| lactose monohydrate | 70.5 mg |

The tablets of the above composition are prepared by homogenization of the calculated quantity of the active ingredient into the powder mixture of lactose monohydrate and maize-starch, or spraying the solution of it in alcohol and/or in a mixture of alcohol : chloroform onto the above powder mixture of lactose and maize-starch (forming the inner phase) in a fluidization granulation equipment.

Then the granulating liquid prepared from a part of the maize-starch is sprayed into the mixture and the fluid bed is solidified with streaming air. The granulation takes place parallel with the drying. The obtained grains are regranulated if necessary, hydrophilic colloidal silicium dioxide, the talcum and the magnesium stearate (forming the outer phase) are admixed, then tablets are pressed from the homogenous mixture.

### Example 2

Tablets of 100 mg total weight are prepared from the following ingredients for each tablet:

| | |
|---|---|
| levonorgestrel | 1.5 mg |
| hydrophilic colloidal silicium dioxide | 0.5 mg |
| talcum | 0.5 mg |
| magnesium stearate | 1.0 mg |
| polivinyl-pirrolidon (Polivinodum K-30) | 2.5 mg |
| croscarmellose sodium | 4.0 mg |
| lactose monohydrate | 40.0 mg |
| microcrystalline cellulose | 50.0 mg |

The tablets of the above given composition are prepared by homogenization of the calculated quantity of the active ingredient into the powder mixture of lactose monohydrate and microcrystalline cellulose, or spraying the solution of it in alcohol and/or in a mixture of alcohol : chloroform onto the above powder mixture of lactose and microcrystalline cellulose (forming the inner phase) in a kneading granulation equipment.

Then the granulating liquid prepared from PVP-K30 is added and the product is granulated by kneading. The wet granulates are regranulated, and dried in a microwave vacuum dryer or in a fluidization equipment. Croscarmellose sodium, hydrophilic colloidal silicium dioxide, talcum and magnesium stearate (forming in the outer phase) are mixed to the above granulates, then 100 mg tablets are pressed from the homogenous mixture.

### Example 3

Tablets of 100 mg total weight are prepared from the following ingredients for each tablet:

| | |
|---|---|
| levonorgestrel | 1.5 mg |
| microcrystalline cellulose | 50.0 mg |
| hydrophilic colloidal silicium dioxide | 0.5 mg |
| pregelatinized starch | 4.0 mg |
| ultra amolipectine | 3.0 mg |
| magnesium stearate | 1.0 mg |
| lactose monohydrate | 40.0 mg |

The above composition of powder mixture is homogenized with the exception of magnesium stearate in a tank or in a container homogenizer, then magnesium stearate is added and the powder mixture is end-homogenized. Then 100 mg tablets are pressed from the homogenous powder mixture.

## Claims

1. Pharmaceutical composition as single application dose, **characterized by** containing 1.5 ± 0.2 mg of levonorgestrel as active ingredient in admixture with known excipients, diluents, flavoring or aromatising agents, stabilizers, as well as formulation-promoting or formulation-providing additives, commonly used in the pharmaceutical practice.

2. Use of 1.5 ± 0.2 mg levonorgestrel for the preparation of a pharmaceutical for emergency contraception.

3. The use as claimed in claim 2, wherein the pharmaceutical is for the administration of a single application dose up to 72 hours of the coitus.

## Patentansprüche

1. Pharmazeutische Zusammensetzung als Einzelverabreichungsdosis, **dadurch gekennzeichnet, dass** sie 1,5 ± 0,2 mg Levonorgestrel als Wirkstoff zusammen mit bekannten Trägerstoffen, Verdünnungsmitteln, Geschmacks- oder Aromastoffen, Stabilisatoren sowie formulierungsunterstützenden oder formulierungsbereitstellenden Zusätzen enthält, die gewöhnlich in der pharmazeutischen Praxis verwendet werden.

2. Verwendung von 1,5 ± 0,2 mg Levonorgestrel zur Herstellung eines Arzneimittels zur Schwangerschaftsverhütung im Notfall.

3. Verwendung wie in Anspruch 2 beansprucht, wobei das Arzneimittel für die Verabreichung einer Einzelverabreichungsdosis bis zu 72 Stunden nach dem Koitus ist.

## Revendications

1. Composition pharmaceutique en tant que dose d'application unique **caractérisée en ce qu'**elle contient 1,5 ± 0,2 mg de lévonorgestrel à titre d'ingrédient actif en addition avec les excipients, diluants, assaisonnants ou aromatisants, stabilisateurs, ainsi que les additifs améliorant la formulation ou les additifs donnant une formulation, connus, utilisés communément dans la pratique pharmaceutique.

2. Utilisation de 1,5 ± 0,2 mg de lévonorgestrel pour la préparation d'une composition pharmaceutique pour la contraception d'urgence.

3. , Utilisation selon la revendication 2 , dans laquelle la composition pharmaceutique est destinée à l'administration d'une dose d'application unique jusqu'à 72 heures suivant le coït.
